# EUROPEAN PATENT SPECIFICATION

(11) **EP 2 588 095 B1**
(45) Date of publication and mention of the grant of the patent: **05.12.2018**
(21) Application number: 11714081.4
(22) Date of filing: 04.04.2011
(51) Int. Cl.: A61K 31/164, A61K 31/455, A61K 31/63, A61K 36/484, A61K 36/61, A61P 17/10

(54) **COMPOSITION FOR THE TREATMENT OF ACNE VULGARIS**
ZUSAMMENSETZUNG ZUR BEHANDLUNG VON AKNE VULGARIS
COMPOSITION POUR LE TRAITEMENT DE L'ACNÉ SIMPLE

(30) Priority: 01.07.2010 GB 201011073
(43) Date of publication of application: 08.05.2013
(73) Proprietor: Lalvani, Kartar Singh, London NW2 7HF (GB)
(72) Inventor: Lalvani, Kartar Singh, London NW2 7HF (GB)
(86) International application number: PCT/GB2011/000518
(87) International publication number: WO 2012/001338

(56) References cited:
- WO-A1-2004/098616
- WO-A1-2005/030157
- CN-A- 1 183 962
- US-A- 6 093 408
- ENSHAIEH S ET AL: "The efficacy of 5% topical tea tree oil gel in mild to moderate acne vulgaris: A randomized, double-blind placebo-controlled study", INDIAN JOURNAL OF DERMATOLOGY, VENEREOLOGY AND LEPROLOGY 20070101 IN, vol. 73, no. 1, 1 January 2007 (2007-01-01), pages 22-25, XP009148255, ISSN: 0378-6323
- SHALITA ALAN R ET AL: "Topical nicotinamide compared with clindamycin gel in the treatment of inflammatory acne vulgaris", INTERNATIONAL JOURNAL OF DERMATOLOGY, vol. 34, no. 6, 1995, pages 434-437, XP000872068, ISSN: 0011-9059
- Anonymous: "Nicotinamide", Wikipedia , 11 September 2015 (2015-09-11), Retrieved from the Internet: URL:https://en.wikipedia.org/wiki/Nicotina mide

## Description

### TECHNICAL FIELD

This invention concerns the composition of a lotion for the treatment of Acne Vulgaris that includes Sodium Sulphacetamide, Niacinamide, D-Panthenol, Tea tree oil, Licorice root extract, and Boric Acid.

### BACKGROUND ART

Sebaceous glands of the dermis secrete sebum. Sebaceous glands are particularly abundant in the face and neck. They have a holocrine mode of secretion, which means that the sebum containing vesicles burst to release sebum at the skin's surface. Sebum has a bactericidal property. Hormones, particularly androgens such as testosterone, stimulate the release of sebum. Levels of testosterone increase during puberty, thereby increasing sebum levels.

### Causes

Acne vulgaris is the active inflammation of sebaceous glands. During homeostatic imbalance, the sebaceous glands may become blocked, producing a white head. When this white head oxidises and dries it becomes a blackhead. Another symptom of acne is pimples or cysts. Factors that cause acne include excess sebum, follicular epidermal hyperproliferation, excess sebum, active Propionibacterium acnes and inflammation.

During the production of excess sebum, acne vulgaris is said to develop further. The level of androgen hormone present in the subject determines sebum production and secretion, as these hormones encourage both production and release.

### Treatments

There are various modes of treatment for acne vulgaris. Treatment is usually topical through absorption of the skin, the rate of which is determined by the thickness of the stratum corneum.

More commonly used for its treatment are Retinoids. They have immune defence properties and are used to treat inflamed skin. They are also used to enhance the penetration of other topical drugs. Retinoids are available in different forms. Tretinoin is one form, which reduces hyperkeratinisation. This would lead to mocrocomedene formation, which is the initial lesion in bacteria. This may also be used to treat photo-damaged skin, and also smoothing out of the skin. When used it is used in a gel or cream form, with concentrations ranging from 0.01% to 0.1%., the cream being used mainly for dry skin and the gel for more oily skin. By incorporating microsponges, irritation is decreased due to slow release of the medication. It also enhances efficacy by target delivery to the sebaceous follicle.

Adaplalene is like Tretinoin, and is also an anti-inflammatory but is stable in sun light, and is less irritating, so does not require the addition of microsponges. Tazarotene is used as it binds to all three RARs that helps the correct effect of the drug to be expressed and also to reduce side effects. Human skin contain two of the three isoforms of the RAR. It can be used alone, or combined with a topical corticosteroid. Isotretinoin is used orally for severe cases of nodulocystic acne vulgaris, which can be normalised after one course. It normalises keratinisation in the sebaceous follicle, decreases the number of sebocytes, therefore reducing sebum synthesis and also a decrease in Propionibacterium acnes, which is the organism that produces inflammation in acne. Orally, the patient is given 0.5-2 mg/kg per day over 15-20 weeks. A lower dose can be used but will be linked with a shorter remission period.

### Prescribed treatments

Antibiotics, in either a topical or systemic form can be used to treat acne vulgaris. The bacterium mentioned above, Propionibacterium acnes release irritants in the form of free fatty acids forming mocrocomedo leading to acne. This bacterium can be suppressed by the use of antibiotics. The most commonly used topical antimicrobials are erythromyocin, clidamycin and benezoyl peroxide. If the disease is more extensive, systemic antibiotics may be used, such as, tetracycline, minocycline, erythromyocin, clindamycin and trimethoprim-sulfamethoxazole. Most Topical antibiotics, such as erythromycin and clindamycin cause side effects such as a burning sensation, dryness, hives, itching, oiliness, peeling and scaling etc.

### Over the counter treatments

On the market, the most common over the counter remedy for acne is a gel or cream containing Benzoyl Peroxide. It is said to be as effective as an antibiotic, or better as it does not cause any antibiotic resistance. For more severe cases, it can be used in combination with the antibiotic erythromyocin. It is fairly harsh, and can cause soreness of the skin. A more comforting alternative is azelaic acid. Salicylic acid may also be used. This is most effective if the fast shedding of hair, which may cause the clogging of pores causing white and black head, causes the acne.

Antibiotics currently used for acne treatment, except Clindamycin are active against aerobic gram positive bacteria only. Clindamycin also has side effects such as burning, itching, dryness, redness, oily skin or skin peeling.

### Natural remedies

There are also a wide variety of herbal remedies that may also be used to treat acne.

Green tea extract when mixed with honeysuckle is referred to as 'pimple tea'. It has antioxidants called polyphenals neutralising the damaging effects of free radicals and toxins, which may be damaging to the skin. Green tea bags may also be applied topically for positive effects.

Aloe Vera is a gentle alternative to other remedies mentioned. It has anti-inflammatory and immune boosting properties. It also reduces scars, related to blemishes. It has 6 antiseptic agents, all of which have antimicrobial activity. It has the ability to penetrate 7 layers of skin and penetrate 4 times faster than water. It can be administered for oral or topical use.

Red clover is taken orally and is a blood cleanser reducing acne breakout. Dandelion root is another natural remedy. It can be taken in the form of a 'tea'. It cleanses the liver, eliminates excess hormones and accelerates elimination of toxins from the body. It includes sources of vitamins A, B, C, D and trace elements.

### DISCLOSURE OF INVENTION

The object of the present invention is to develop a synergistic composition for the treatment of Acne that could overcome the disadvantages of the conventional acne treatment (both prescribed and OTC) and provide a better alternative.

The product can include, by weight, 3% to 7% D-Panthenol, 1% to 15% Niacinamide, 2% to 30 % of Sulphacetamide Sodium, 2% to 10% Tea tree oil, 2% to 10% Licorice root extract, and around 1% Boric Acid,

The preferred concentrations according to weight are : 3% to 7% Niacinamide, 1% to 5% D-Panthenol, 5% to 7% sulphacetamide sodium, 3% to 7% Tea tree oil, 3% to 7% Licorice root extract, 1% Boric Acid.

Sodium suphacetamide has commonly been used in eye drops for 60 years with a good safety record. For the last 60 years the eye drops have contained sodium sulphacetamide in a concentration of 10% to 30% by weight in an aqueous solution. The preferred strength of sulphacetamide is 5% to 7% by weight, because at this strength it is not known to show any side effects and is an effective bacteriostatic and also bactericidal against the anaerobe propionicbacterium acnes. Sulphacetamide is a sulfonamide antibiotic. The sulfonamides are synthetic bacteriostatic antibiotics with a wide spectrum against most gram-positive and many gram-negative organisms. However, many strains of an individual species may be resistant. Sulfonamides inhibit multiplication of bacteria by acting as competitive inhibitors of *p*-aminobenzoic acid in the folic acid metabolism cycle. Bacterial sensitivity is the same for the various sulfonamides, and resistance to one sulfonamide indicates resistance to all. Most sulfonamides are readily absorbed orally. However, parenteral administration is difficult, since the soluble sulfonamide salts are highly alkaline and irritating to the tissues. High levels are achieved in pleural, peritoneal, synovial, and ocular fluids. Their antibacterial action is inhibited by pus.

The inclusion of Niacinamide in the formula is due to its anti-acne action. Topical niacinamide has been shown to provide potent anti-inflammatory activity in the treatment of acne vulgaris. Many practitioners use the treatment for its combination of efficacy and unlikeliness of causing bacterial resistance.

The inclusion of D-Panthenol in the formula is because of the following properties : it improves and increases the humidity properties of the skin (a moisturising effect), and also makes dry skin softer and more elastic. It has an anti-inflammatory effect and soothes irritated skin.

Boric Acid is optimally included in the formulation in a proportion of around 1% according to weight.

Tea tree oil is an essential oil obtained by steam distillation of the leaves of Melaleuca alternifolia, a plant native to Australia. Tea tree oil contains terpinen-4-ol that is thought to be responsible for most of tea tree oil's antimicrobial activity. Tea tree oil is an antiseptic, which kills germs and bacteria, acting as an antibacterial. Because tea tree oil can kill bacteria, applying topical tea tree oil to acne lesions has been thought to kill Propionibacterium acnes, the skin-dwelling bacteria that is involved in causing acne. It has the ability to reduce the number of breakouts, but cannot cure. It is relatively strong and can cause irritation, so is usually combined with a cream. It is said to be an alternative to Benzoyl Peroxide, but has a much slower action.

Licorice root extract is included for post healing/ reduction in scarring. Licorice root has anti-inflammatory properties. When applied topically it soothes the skin and also inhibits the production of irritants.

The following examples are some of the possible forms of the invention. These examples are not to be construed as limiting the invention in scope or spirit, as possible modifications will be apparent from the disclosure to those skilled in this art.

### Example one:

Sodium Sulphacetamide - 7% by weight
Niacinamide - 4% by weight
Tea tree oil - 5% by weight
Licorice root extract - 5% by weight
D-Panthenol - 2% by weight
Boric Acid - 1% by weight

### Example two:

Sodium Sulphacetamide - 7% by weight
Niacinamide - 4% by weight
Tea tree oil - 4% by weight
Licorice root extract - 4% by weight
D-Panthenol - 3% by weight
Boric Acid - 1% by weight

### Example three:

Sodium Sulphacetamide - 7% by weight
Niacinamide - 5% by weight
Tea tree oil - 5% by weight
Licorice root extract - 4% by weight
D-Panthenol - 4% by weight
Boric Acid - 1% by weight

### Example four:

Sodium Sulphacetamide - 8% by weight
Niacinamide - 4% by weight
Tea tree oil - 4% by weight
Licorice root extract - 3% by weight
D-Panthenol - 5% by weight
Boric Acid - 1% by weight

### Example five:

Sodium Sulphacetamide - 10% by weight
Niacinamide - 4% by weight
Tea tree oil - 3% by weight
Licorice root extract - 3% by weight
D-Panthenol - 3% by weight
Boric Acid - 1% by weight

The composition of the present invention not only shows good clinical efficacy in the treatment of Acne but also enables the prevention of the reoccurrence within a reasonable period of time. The composition according to this invention is effective against active Propionibacterium acnes.

### Means of administration

The fomulations are to be used in the form of a lotion, whose constituents and proportions can be adapted to common practice and need. Other products of this kind are usually in a cream or gel form, that are in combined phases, and contain oil, which can exacerbate the sensitive skin in Acne Vulgaris.

Unusually, this invention requires only a single aqueous phase, with water making up the rest of the lotion. Due to the constituents used, no other carrier or excipient is needed, because the active constituents are stable in water. This provides therapeutic advantages by avoiding the exacerbation of the skin that can inadvertently occur with other prepations.

### INDUSTRIAL APPLICABILITY

According to this invention, there is a synergistic composition for the treatment of Acne Vulgaris that could overcome the disadvantages of the conventional acne treatment (both prescribed and OTC) and provide a better alternative, or the use of that composition in the manufacture of a medicament for that purpose. In its optimal embodiment the composition consists by weight, and in the form of a lotion, the rest of which is made up of water, of : 3% to 7% of D-Panthenol, 3% to 7% of Niacinamide, 5% to 7% of Sulphacetamide Sodium, 3% to 7% Tea tree oil, 3% to 7% Licorice root extract, and 1% Boric Acid.

## Claims

1. A composition for the treatment of Acne Vulgaris that : (a) is administered in a single aqueous phase, in the form of a lotion, the rest of which is made up solely of water, and which does not include the use of additional oil, and that (b) consists by weight of : 3% to 7% of D-Panthenol, 3% to 7% of Niacinamide, 5% to 7% of Sulphacetamide Sodium, 3% to 7% Tea tree oil, 3% to 7% Licorice root extract, and 1% Boric Acid, and (c) in which the total weight of D-Panthenol, Niacinamide, Sulphacetamide Sodium, and Boric Acid equals or exceeds 12% of the composition.

2. A composition for the treatment of Acne Vulgaris that : (a) is administered in a single aqueous phase, in the form of a lotion, the rest of which is made up solely of water, and which does not include the use of additional oil, and that (b) consists by weight of : 3% to 7% of D-Panthenol, 3% to 7% of Niacinamide, 5% to 7% of Sulphacetamide Sodium, 3% to 7% Tea tree oil, 3% to 7% Licorice root extract, and 1% Boric Acid.

3. A composition for the treatment of Acne Vulgaris that : (a) is administered in a single aqueous phase, in the form of a lotion, the rest of which is made up solely of water, and which does not include the use of additional oil, and that (b) consists by weight of : 3% to 7% of D-Panthenol, 1% to 15% of Niacinamide, 2% to 30% of Sulphacetamide Sodium, 2% to 10% Tea tree oil, 2% to 10% Licorice root extract, and 1% Boric Acid.

4. A composition for the treatment of Acne Vulgaris that : (a) is administered in a single aqueous phase, in the form of a lotion, the rest of which is made up solely of water, and which does not include the use of additional oil, and that (b) consists of : D-Panthenol, Niacinamide, Sulphacetamide Sodium, Tea tree oil, Licorice root extract, and Boric Acid, in which Sulphacetamide Sodium is the greatest active constituent according to weight.

5. A composition for the treatment of Acne Vulgaris that : (a) is administered in a single aqueous phase, in the form of a lotion, the rest of which is made up solely of water, and which does not include the use of additional oil, and that (b) consists of : D-Panthenol, Niacinamide, Sulphacetamide Sodium, Tea tree oil, Licorice root extract, and Boric Acid.

6. A composition for the treatment of Acne Vulgaris that : (a) is administered in the form of a lotion, the rest of which is made up of water, but which can include the use of additional oil, and that (b) consists by weight of : 3% to 7% of D-Panthenol, 3% to 7% of Niacinamide, 5% to 7% of Sulphacetamide Sodium, 3% to 7% Tea tree oil, 3% to 7% Licorice root extract, and 1% Boric Acid.

7. A composition for the treatment of Acne Vulgaris that : (a) is administered in the form of a lotion, the rest of which is made up of water, but which can include the use of additional oil, and that (b) consists of : D-Panthenol, Niacinamide, Sulphacetamide Sodium, Tea tree oil, Licorice root extract, and Boric Acid.

8. A composition for the treatment of Acne Vulgaris that : (a) is administered in in the form of a lotion, and that (b) consists by weight of : 3% to 7% of D-Panthenol, 3% to 7% of Niacinamide, 5% to 7% of Sulphacetamide Sodium, 3% to 7% Tea tree oil, 3% to 7% Licorice root extract, and 1% Boric Acid, and (c) in which the total weight of D-Panthenol, Niacinamide, Sulphacetamide Sodium, and Boric Acid equals or exceeds 12% of the composition.

9. A composition for the treatment of Acne Vulgaris that : (a) is administered in the form of a lotion, and that (b) consists of : D-Panthenol, Niacinamide, Sulphacetamide Sodium, Tea tree oil, Licorice root extract, and Boric Acid.

10. A composition for the treatment of Acne Vulgaris that comprises in the form of a lotion of : D-Panthenol, Niacinamide, Sulphacetamide Sodium, Tea tree oil, Licorice root extract, and Boric Acid.

11. A claim according to any one of the preceding claims in which the composition is not used in the form of a lotion.

12. A claim according to any one of claims 1 to 11, in which Vitamin B3 is used instead of Niacinamide.

13. A claim according to any one of claims 1 to 11, in which Vitamin B5 is used instead of D-Panthenol.

14. The use in the manufacture of a medicament using a composition as described in any one of the preceding claims.

## Patentansprüche

1. Zusammensetzung zur Behandlung von Akne vulgaris, die: (a) in einer einzigen wässrigen Phase in Form einer Lotion verabreicht wird, wobei der Rest von ihr ausschließlich aus Wasser besteht, und die nicht die Verwendung von zusätzlichem Öl einschließt, und die (b) aus folgenden Bestandteilen, bezogen auf Gewicht, besteht: 3% bis 7% D-Panthenol, 3% bis 7% Niacinamid, 5% bis 7% Sulfacetamid-Natrium, 3% bis 7% Teebaumöl, 3% bis 7% Süßholzwurzelextrakt und 1% Borsäure, und (c) worin das Gesamtgewicht von D-Panthenol, Niacinamid, Sulfacetamid-Natrium und Borsäure 12% der Zusammensetzung beträgt oder übersteigt.

2. Zusammensetzung zur Behandlung von Akne vulgaris, die : (a) in einer einzigen wässrigen Phase in Form einer Lotion verabreicht wird, wobei der Rest von ihr ausschließlich aus Wasser besteht, und die nicht die Verwendung von zusätzlichem Öl einschließt, und die (b) aus Folgendem, bezogen auf Gewicht, besteht: 3% bis 7% D-Panthenol, 3% bis 7% Niacinamid, 5% bis 7% Sulfacetamid-Natrium, 3% bis 7% Teebaumöl, 3% bis 7% Süßholzwurzelextrakt und 1% Borsäure.

3. Zusammensetzung zur Behandlung von Akne vulgaris, die : (a) in einer einzigen wässrigen Phase in Form einer Lotion verabreicht wird, wobei der Rest von ihr ausschließlich aus Wasser besteht, und die nicht die Verwendung von zusätzlichem Öl einschließt, und die (b) aus Folgendem, bezogen auf Gewicht, besteht: 3% bis 7% D-Panthenol, 1% bis 15% Niacinamid, 2% bis 30% Sulfacetamid-Natrium, 2% bis 10% Teebaumöl, 2% bis 10% Süßholzwurzelextrakt und 1% Borsäure.

4. Zusammensetzung zur Behandlung von Akne vulgaris, die : (a) in einer einzigen wässrigen Phase in Form einer Lotion verabreicht wird, wobei der Rest von ihr ausschließlich aus Wasser besteht, und die nicht die Verwendung von zusätzlichem Öl einschließt, und die (b) aus Folgendem besteht: D-Panthenol, Niacinamid, Sulfacetamid-Natrium, Teebaumöl, Süßholzwurzelextrakt und Borsäure, wobei Sulfacetamid-Natrium der größte aktive Bestandteil nach Gewicht ist.

5. Zusammensetzung zur Behandlung von Akne vulgaris, die : (a) in einer einzigen wässrigen Phase in Form einer Lotion verabreicht wird, wobei der Rest von ihr ausschließlich aus Wasser besteht, und die nicht die Verwendung von zusätzlichem Öl einschließt, und die (b) aus Folgendem besteht: D-Panthenol, Niacinamid, Sulfacetamid-Natrium, Teebaumöl, Süßholzwurzelextrakt und Borsäure.

6. Zusammensetzung zur Behandlung von Akne vulgaris, die : (a) in Form einer Lotion verabreicht wird, wobei der Rest von ihr aus Wasser besteht, aber die die Verwendung von zusätzlichem Öl einschließen kann und die (b) aus Folgendem, bezogen auf Gewicht, besteht: 3% bis 7% D-Panthenol, 3% bis 7% Niacinamid, 5% bis 7% Sulfacetamid-Natrium, 3% bis 7% Teebaumöl, 3% bis 7% Süßholzwurzelextrakt und 1% Borsäure.

7. Zusammensetzung zur Behandlung von Akne vulgaris, die : (a) in Form einer Lotion verabreicht wird, wobei der Rest von ihr aus Wasser besteht, aber die die Verwendung von zusätzlichem Öl einschließen kann, und die (b) besteht aus: D-Panthenol, Niacinamid, Sulfacetamid-Natrium, Teebaumöl, Süßholzwurzelextrakt und Borsäure.

8. Zusammensetzung zur Behandlung von Akne vulgaris, die : (a) in Form einer Lotion verabreicht wird, und die (b) aus Folgendem, bezogen auf Gewicht, besteht: 3% bis 7% D-Panthenol, 3% bis 7% Niacinamid, 5% bis 7% Sulfacetamid-Natrium, 3% bis 7% Teebaumöl, 3% bis 7% Süßholzwurzelextrakt und 1% Borsäure, und (c) wobei das Gesamtgewicht von D-Panthenol, Niacinamid, Sulfacetamid-Natrium und Borsäure 12% der Zusammensetzung beträgt oder übersteigt.

9. Zusammensetzung zur Behandlung von Akne vulgaris, die: (a) in Form einer Lotion verabreicht wird, und die (b) aus Folgendem besteht: D-Panthenol, Niacinamid, Sulfacetamid-Natrium, Teebaumöl, Süßholzwurzelextrakt und Borsäure.

10. Zusammensetzung zur Behandlung von Akne vulgaris, die in Form einer Lotion zusammengesetzt ist aus: D-Panthenol, Niacinamid, Sulfacetamid-Natrium, Teebaumöl, Süßholzwurzelextrakt und Borsäure.

11. Anspruch nach einem der vorhergehenden Ansprüche, wobei die Zusammensetzung nicht in Form einer Lotion verwendet wird.

12. Anspruch nach einem der Ansprüche 1 bis 11, wobei Vitamin B3 anstelle von Niacinamid verwendet wird.

13. Anspruch nach einem der Ansprüche 1 bis 11, wobei Vitamin B5 anstelle von D-Panthenol verwendet wird.

14. Verwendung bei der Herstellung eines Medikaments unter Verwendung einer Zusammensetzung, wie sie in einem der vorhergehenden Ansprüche beschrieben ist.

## Revendications

1. Composition pour le traitement de l'acné vulgaire qui : (a) est administrée dans une phase aqueuse unique, sous la forme d'une lotion, dont le reste est exclusivement constitué d'eau, et qui ne comprend pas l'utilisation d'huile supplémentaire, et qui (b) est constituée, en poids, de : 3 % à 7 % de D-panthénol, 3 % à 7 % de niacinamide, 5 % à 7 % de sulfacétamide de sodium, 3 % à 7 % d'huile d'arbre à thé, 3 % à 7 % d'extrait de racine de réglisse et 1 % d'acide borique, et (c) dans laquelle le poids total de D-panthénol, niacinamide, sulfacétamide de sodium et acide borique est égal ou supérieur à 12 % de la composition.

2. Composition pour le traitement de l'acné vulgaire qui : (a) est administrée dans une phase aqueuse unique, sous la forme d'une lotion, dont le reste est exclusivement constitué d'eau, et qui ne comprend pas l'utilisation d'huile supplémentaire, et qui (b) est constituée, en poids, de : 3 % à 7 % de D-panthénol, 3 % à 7 % de niacinamide, 5 % à 7 % de sulfacétamide de sodium, 3 % à 7 % d'huile d'arbre à thé, 3 % à 7 % d'extrait de racine de réglisse et 1 % d'acide borique.

3. Composition pour le traitement de l'acné vulgaire qui : (a) est administrée dans une phase aqueuse unique, sous la forme d'une lotion, dont le reste est exclusivement constitué d'eau, et qui ne comprend pas l'utilisation d'huile supplémentaire, et qui (b) est constituée en poids de : 3 % à 7 % de D-panthénol, 1 % à 15 % de niacinamide, 2 % à 30 % de sulfacétamide de sodium, 2 % à 10 % d'huile d'arbre à thé, 2 % à 10 % d'extrait de racine de réglisse et 1 % d'acide borique.

4. Composition pour le traitement de l'acné vulgaire qui : (a) est administrée dans une phase aqueuse unique, sous la forme d'une lotion, dont le reste est exclusivement constitué d'eau, et qui ne comprend pas l'utilisation d'huile supplémentaire, et qui (b) est constituée de : D-panthénol, niacinamide, sulfacétamide de sodium, huile d'arbre à thé, extrait de racine de réglisse et acide borique, dans laquelle le sulfacétamide de sodium est le constituant actif le plus abondant en poids.

5. Composition pour le traitement de l'acné vulgaire qui : (a) est administrée dans une phase aqueuse unique, sous la forme d'une lotion, dont le reste est exclusivement constitué d'eau, et qui ne comprend pas l'utilisation d'huile supplémentaire, et qui (b) est constituée de : D-panthénol, niacinamide, sulfacétamide de sodium, huile d'arbre à thé, extrait de racine de réglisse et acide borique.

6. Composition pour le traitement de l'acné vulgaire qui : (a) est administrée sous la forme d'une lotion, dont le reste est constitué d'eau, mais qui peut comprendre l'utilisation d'huile supplémentaire, et qui (b) est constituée en poids de : 3 % à 7 % de D-panthénol, 3 % à 7 % de niacinamide, 5 % à 7 % de sulfacétamide de sodium, 3 % à 7 % d'huile d'arbre à thé, 3 % à 7 % d'extrait de racine de réglisse et 1 % d'acide borique.

7. Composition pour le traitement de l'acné vulgaire qui : (a) est administrée sous la forme d'une lotion, dont le reste est constitué d'eau, mais qui peut comprendre l'utilisation d'huile supplémentaire, et qui (b) est constituée de : D-panthénol, niacinamide, sulfacétamide de sodium, huile d'arbre à thé, extrait de racine de réglisse et acide borique.

8. Composition pour le traitement de l'acné vulgaire qui : (a) est administrée sous la forme d'une lotion, et qui (b) est constituée en poids de : 3 % à 7 % de D-panthénol, 3 % à 7 % de niacinamide, 5 % à 7 % de sulfacétamide de sodium, 3 % à 7 % d'huile d'arbre à thé, 3 % à 7 % d'extrait de racine de réglisse et 1 % d'acide borique, et (c) dans laquelle le poids total de D-panthénol, niacinamide, sulfacétamide de sodium et acide borique est égal ou supérieur à 12 % de la composition.

9. Composition pour le traitement de l'acné vulgaire qui : (a) est administrée sous la forme d'une lotion, et qui (b) est constituée de : D-panthénol, niacinamide, sulfacétamide de sodium, huile d'arbre à thé, extrait de racine de réglisse et acide borique.

10. Composition pour le traitement de l'acné vulgaire qui comprend, sous la forme d'une lotion, les composants suivants : D-panthénol, niacinamide, sulfacétamide de sodium, huile d'arbre à thé, extrait de racine de réglisse et acide borique.

11. Revendication selon l'une quelconque des revendications précédentes dans laquelle la composition n'est pas utilisée sous la forme d'une lotion.

12. Revendication selon l'une quelconque des revendications 1 à 11, dans laquelle la vitamine B3 est utilisée à la place du niacinamide.

13. Revendication selon l'une quelconque des revendications 1 à 11, dans laquelle la vitamine B5 est utilisée à la place du D-panthénol.

14. Utilisation dans la fabrication d'un médicament utilisant une composition telle que décrite dans l'une quelconque des revendications précédentes.
